# EUROPÄISCHE PATENTSCHRIFT

(11) **EP 1 273 905 B1**
(45) Veröffentlichungstag und Bekanntmachung des Hinweises auf die Patenterteilung: **21.09.2005**
(21) Anmeldenummer: 01116410.0
(22) Anmeldetag: 06.07.2001
(51) Int. Cl.: G01N 21/55, G01N 33/34, B41M 5/03

(54) **Verfahren zum Bestimmen der Papierqualität für autotypischen Rasterdruck**
Method for determination of quality of paper for printing process
Méthode pour la détermination de la qualité de papier pour des procédés d'imprimer

(43) Veröffentlichungstag der Anmeldung: 08.01.2003
(73) Patentinhaber: Nordland Papier GmbH, 26892 Dörpen (DE)
(72) Erfinder: Carl, Gerd, 52152 Simmerath (DE)
(74) Vertreter: Glawe, Delfs, Moll

(56) Entgegenhaltungen:
- WO-A-00/39749
- JP-A- 6 286 325
- US-A- 4 541 273
- US-A- 6 153 038

## Beschreibung

Die Erfindung betrifft ein Verfahren zum Bestimmen der Papierqualität für autotypischen Rasterdruck, bei dem ein fein verteiltes Muster von geometrischen Figuren auf das Papier aufgebracht wird, das Papier beleuchtet wird und das vom Papier reflektierte und gestreute Licht beobachtet wird.

Beim Drucken tritt das Problem auf, daß sich Grautöne nur schlecht wiedergeben lassen. Um trotzdem Drucke guter Qualität mit abgestuften Helligkeitstönen erzeugen zu können, bedient man sich daher in weit verbreitetem Umfang der Technik des sog. autotypischen Rasterdrucks. Dabei wird das Bild nicht mehr durch kontinuierlich Helligkeitsübergänge bestimmt. Vielmehr wird das Bild aus einem feinverteilten Muster von geometrischen Figuren aufgebaut, die meistens Punkte sind. Diese Punkte sind bei einer weitverbreiteten Drucktechnik regelmäßig rasterförmig angeordnet. Je nach Helligkeitswert an einer bestimmten Stelle des zu druckenden Bildes werden diese Punkte größer oder kleiner gemacht. Sind die Punkte genügend dicht angeordnet und betrachtet man das Druckbild aus einer genügenden Entfernung, so nimmt man die einzelnen Punkte nicht mehr wahr, sondern erhält den Eindruck eines Bildes mit verschiedenen Grautönen bzw., wenn Punkte verschiedener Farben verwendet werden, von verschiedenen Farbtönen. Bei gewissen Anwendungen können die Punkte auch unregelmäßig angeordnet sein, wobei auch der Abstand bzw. die Anzahl der Punkte bestimmt, welche Grautöne oder Farbtöne vom Beobachter wahrgenommen werden.

Die Größe eines solchen Punktes kann entsprechend der Helligkeit des Bildpunktes berechnet werden. Dabei muß aber berücksichtigt werden, daß der Punkt praktisch eine größere Fläche einnehmen wird bzw. das vom Auge wahrgenommene Druckbild mehr beeinflußt, als dies theoretisch der Fall sein müßte. Ein Problem der Drucktechnik, das von der Papierqualität der Drucktechnik und anderen Faktoren abhängt, besteht darin, daß der Punkt beim Drucken physisch größer sein wird, als dies beabsichtigt ist. Insbesondere bei Drucken hoher Qualität muß dies natürlich berücksichtigt werden. Für solche Drucke benutzt man normalerweise beschichtetes Papier, bei dem durch entsprechende Oberflächengestaltung dafür gesorgt wird, daß der Punkt beim Drucken nicht wesentlich größer wird, als dies beabsichtigt ist. Hier tritt aber ein anderes Problem der scheinbaren Vergrößerung des Punktes auf. Die Beschichtung ist nicht vollständig lichtundurchlässig. Das Licht dringt vielmehr bis zu einer gewissen Tiefe in die Beschichtung und in die darunterliegende Farbschicht ein und wird dann zurückgestreut. Das zurückgestreute Licht erreicht aber nicht immer das Auge des Beobachters. Dringt ein Lichtstrahl sehr nahe am Rand eines Punktes in die Beschichtung unter einem schrägen Winkel ein, kann er unter den Punkt gestreut werden und nicht mehr aus dem Papier heraustreten. Lichtstrahlen, die nahe beim Punkt eintreten, treten daher teilweise nicht wieder aus der Beschichtung heraus, so daß der Punkt größer erscheint, als er tatsächlich ist; der Rand wird "verschmiert", zeigt einen Schatten (was als Lichthof bezeichnet wird).

Es existieren mathematische Modelle, wie diese Punktvergrößerung bestimmt werden kann (Dissertation "Dot Gain in Colour Halftones" von Stefan Gustavson, Linköping, September 1997). Diese tatsächliche oder scheinbare Vergrößerung der Punkte kann aufgrund dieser mathematischen Modelle oder aber aufgrund von optischer Beobachtung des fertigen Drucks berücksichtigt werden, so daß theoretisch einwandfreie Drucke hergestellt werden können. Dies ist aber eine rein theoretische Folgerung. Technisch ist das nicht umsetzbar, da keine der jeweiligen Stelle entsprechend angepaßten Druckpunkte erzeugt werden können.

Es hat sich aber gezeigt, daß auch der optische Oberflächeneindruck eines beschichteten Papiers nicht vollkommen gleichmäßig ist. So kann das Papier einen ungleichmäßigen Glanz aufweisen, was selbstverständlich zu einer ungleichmäßigen Intensität des reflektierten oder gestreuten Lichtes bei Betrachtung des Druckes führt. Die Helligkeit oder weiße Farbe der Beschichtung können unterschiedlich sein, was an einer gewissen Schwärzung, sichtbaren Fasern des Papiers, die durch die Beschichtung nicht genügend abgedeckt sind und/oder einer ungleichmäßigen Verteilung von optischen Aufhellern bedingt sein kann. Auch die Deckkraft oder Lichtundurchlässigkeit der Beschichtung kann ungleichmäßig sein, was mit den vorhergenannten Ursachen zusammenhängen kann, so daß Lichtstrahlen unterschiedlich tief in die Papierstruktur eindringen. Auch die Dicke der Beschichtung kann lokal variieren, was zu unterschiedlicher Streuung in der Beschichtung und auch zu ungleichmäßiger Vergrößerung des wahrgenommenen Bildes des Punktes führt.

Alle diese Unperfektheiten des Papiers führen zu unterschiedlichen Punktvergrößerungen. Man wird sie in mehr oder weniger starkem Ausmaß bei Betrachtung des Drucks feststellen können, wenn dieser fertiggestellt ist. Von diesem Stand der Technik (siehe z.B. WO 00/39749) geht die vorliegende Erfindung aus, wobei es selbstverständlich unbefriedigend ist, daß die mangelnde Qualität des Papiers erst nach der aufwendigen Herstellung des Drucks erkannt werden kann. Insbesondere kann natürlich ein Papierhersteller in den von ihm gelieferten Papierrollen nicht zwischendurch zu Testzwecken solche Drucke anbringen.

Die Aufgabe der Erfindung besteht in der Schaffung eines Verfahrens der eingangs genannten Art, mit dem die Qualität des Papiers vor dem Druck und ohne einen solchen Druck einfach und zuverlässig festgestellt werden kann.

Die erfindungsgemäße Lösung besteht darin, daß das Muster auf einem optisch transparenten flächigen Element vorgesehen ist, dieses mit der Seite, auf der sich das Muster befindet, mit dem Papier flächig in Berührung gebracht wird, und daß das Papier gegen das flächige Element gedrückt wird.

Es ist zwar bekannt, einen Zwischenträger mit einem Druck zu versehen und diesen dann auf den zu bedruckenden Gegenstand zu übertragen (US 6 153 038). Dabei wird aber der Gegenstand, im vorliegenden Falle wäre das das Papier, bedruckt, so daß wie beim anderen Verfahren des Standes der Technik (WO 00/39749) anschließend ein bedrucktes Papier vorliegen würde, das nicht weiter verwendet werden kann.

Es findet also eine Bestimmung der Papierqualität statt, ohne daß ein Druck ausgeführt werden muß. Es ist zwar rein theoretisch in der genannten Dissertation von Gustavson vorgeschlagen worden, ein Raster auf das Papier abzubilden und das zurückgestreute Licht durch das Raster zu beobachten, um damit die scheinbare Vergrößerung der Punkte zu bestimmen. Das Raster berührt dabei das Papier nicht. Hier handelt es sich aber, wie der Autor selber zugibt, um rein theoretische Überlegungen, die nicht experimentell überprüft wurden. Man muß sich hier fragen, wie der Effekt eines unter einen Farbpunkt diffundierten und somit nicht mehr zu beobachtenden Lichtstrahls simuliert werden soll, wenn kein einziger Lichtstrahl, der aus der Beschichtung wieder austritt tatsächlich an der Oberfläche dieser Beschichtung absorbiert wird. Außerdem dient dieses theoretisch vorgeschlagene Verfahren nicht zur Bestimmung der Qualität von Papier, insbesondere von Qualitätsschwankungen, sondern zur Überprüfung von mathematischen Modellen der erwähnten scheinbaren Vergrößerung der Punkte. In der Dissertation ist auch ein Verfahren erwähnt, bei dem ein Film, auf dem die entsprechenden Punkte erzeugt sind, auf das Papier gelegt wird. Dieses Verfahren, mit dem wieder die Punktverbreiterung untersucht werden soll, leidet jedoch nach Angaben des Verfassers der genannten Dissertation darunter, daß Oberflächenreflexionen auftreten, die bei einem normalen Druck nicht vorhanden sind, wodurch das Ergebnis verfälscht wird. Außerdem wurde dieses Verfahren offenbar nur zur theoretischen Untersuchung der Punktverbreiterungsmechanismen, nicht aber zur Bestimmung der Papierqualität verwendet.

Ein ähnliches Verfahren ist beschrieben im Zwischenbericht zum AiF-Forschungsprojekt 12395N des Instituts für Papierfabrikation der Technischen Universität Darmstadt. Auch dort soll aber lediglich Lichtstreuung und -absorption des Papiers auf die Bildwiedergabe gedruckter Rasterflächen untersucht werden, also die obigen Effekte untersucht und überprüft werden. Das damit auch die Papierqualität, insbesondere auch Ungleichmäßigkeiten in der Papierqualität, was den Druck besonders stark negativ beeinflussen würde, untersucht werden können, ist der Entgegenhaltung nicht zu entnehmen. Die Untersuchung der Universität Darmstadt bezieht sich auf den generellen Effekt des Lichtfangphänomens, währenddessen unsere Erfindung die Variation mißt und sichtbar macht. Das Forschungsprojekt dient der erstmaligen praktischen Erforschung der mathematischen Modelle zu generellen optischen Eigenschaften verschiedener Rohstoffe.

Erfindungsgemäß wurde nun herausgefunden, daß auch ohne die bisher notwendigen Probedrucke auf ganz einfache Weise die Papierqualität vor dem Druck bestimmt werden kann. Es treten die beim Stand der Technik bei dem aufgelegten Film aufgetretenen Probleme wegen Reflexion an zusätzlichen Flächen nicht auf bzw. haben keinen Einfluß, da bei vollständig gleichförmigem photographischem Film oder dergleichen die Reflexionsverhältnisse überall die gleichen sind. Da keine Absolutwerte für die Punktvergrößerungen bestimmt werden sollen, arbeitet das Verfahren trotz der in der Dissertation genannten Vorurteile einwandfrei und einfach sowie genau. Die Probleme des Standes der Technik treten vermutlich auch deswegen bei dem erfindungsgemäßen Verfahren nicht auf, weil das Papier sehr gleichförmig gegen das optisch transparente flächige Element gedrückt wird.

Das Muster auf diesem optisch transparentem flächigen Element ist gleichförmig. Ist die Papierqualität über den Bereich dieses flächigen Elementes unterschiedlich, wird man von Auge sofort Unterschiede in der Helligkeit oder aber bei farbigen Punkten möglicherweise auch in der Farbe erkennen. Auf diese Weise erkennt man Fehler des Papiers wesentlich besser, als wenn ein Bild darauf gedruckt wird, das von sich aus schon unterschiedliche Helligkeitsabstufungen aufweist und durch die Punktübertragung des Druckprozesses beeinflußt ist.

Durch die Erfindung wird also in überraschend einfacher Weise ein sehr genaues und empfindliches Verfahren geschaffen, um die Papierqualität vor dem Druck zu überprüfen. Das Verfahren ist dabei nicht nur bei dem beschichteten Papier, sondern auch bei Naturpapier anwendbar.

Das Papier kann durch ein gummielastisches Andrückelement gegen das transparente flächige Element gedrückt werden. Bei einer anderen vorteilhaften Ausführungsform wird das Papier durch eine mit einer Druckdifferenz beaufschlagte Membrane gegen das transparente flächige Element gedrückt. Die Druckdifferenz kann dabei durch eine Druckgasquelle und/oder einen Unterdruck bewirkt werden.

Das transparente flächige Element kann im wesentlichen eben sein. Hier können dann einzelne Blätter auf das flächige Element gelegt werden und untersucht werden, oder die Vorrichtung kann mit unterschiedlichen Stellen einer Papierbahn in Kontakt gebracht werden, um dort die Papierqualität zu bestimmen. Bei einer besonders vorteilhaften Ausführungsform ist aber das transparente flächige Element eine transparente rotierende Walze, in der eine oder mehrere Lichtquellen und lichtempfindliche Elemente angeordnet sind, und das Papier wird durch eine weitere Walze mit gummielastischer Oberfläche gegen die durchsichtige Walze gedrückt. Dabei erfolgt die Beobachtung des gestreuten Lichts mit CCD-Elementen, die natürlich auch bei den im wesentlichen ebenen transparenten flächigen Elementen Anwendung finden können. Die Anordnung mit der transparenten rotierenden Walze ermöglicht es einerseits, eine laufende Papierbahn z. B. bei der Produktion laufend zu überwachen. Dazu braucht nicht die ganze Papierbahn untersucht zu werden, sondern z. B. ein nur ungefähr 5 cm breiter Streifen, da so der Zylinder keine besonders hohen Kosten verursacht. Da die Papierqualität insbesondere eine Funktion der Stoffzusammensetzung ist, kann durch die Messung in einem verhältnismäßig kleinen Streifen Information über die gesamte Breite des Papiers indirekt erhalten werden. Der Durchmesser der transparenten rotierenden Walze ist dabei zweckmäßigerweise kleiner als die Breite, da sonst die Walze bei hohen Geschwindigkeiten instabil laufen könnte. Die transparente rotierende Walze kann aber auch ähnlich wie bei einem Telefaxgerät mit Originaleinzug für die Untersuchung von Einzelblättern verwendet werden.

Wichtig ist es, daß die Punkte fest gegen das Papier gedrückt werden und zwischen Punkten und Papier kein Abstand besteht, da sonst die Punkte unterstrahlt werden.

Als besonders zweckmäßig hat es sich erwiesen, daß die geometrischen Figuren, d.h. im allgemeinen die Punkte ungefähr 15-70% der gesamten Beobachtungsfläche, insbesondere ungefähr 20-50% dieser Beobachtungsfläche bedecken. Auf diese Weise erhält man besonders stark ausgebildete Kontraste bei bereits geringen Unterschieden in der Papier- bzw. Beschichtungsqualität. Zweckmäßigerweise sind die geometrischen Figuren schwarz, da dann Helligkeitsunterschiede besonders deutlich zu sehen sind. Wenn stattdessen geometrische Figuren, insbesondere Punkte unterschiedlicher Farben verwendet werden, so wird das Bild bei schwankender Papier- bzw. Beschichtungsqualität auch Farbschattierungen aufweisen.

Wie bereits erwähnt ist es möglich, beim autotypischen Rasterdruck die Punkte unregelmäßig anzuordnen, wobei der Abstand dieser Punkte den Helligkeitswert möglicherweise zusammen mit der Punktgröße bestimmt. Für das erfindungsgemäße Verfahren ist es aber besonders zweckmäßig, wenn die Punkte regelmäßig gerastert sind. Als besonders zweckmäßig hat es sich erwiesen, wenn man ungefähr 40 bis 100 geometrische Figuren, d.h. Punkte bzw. Linien von Punkten pro Zentimeter vorsieht.

Bei einer vorteilhaften Ausführungsform ist das transparente flächige Element eine Folie oder ein photographischer Film, die besonders einfach mit dem Raster von geometrischen Figuren bzw. Punkten zu versehen sind. Diese Folie oder der photographische Film werden dann durch eine transparente Glas-oder Kunststoffplatte (bzw. die transparente Walze) abgestützt, wenn das Papier durch den Unterdruck und die Membran (bzw. durch die gummielastische Walze) gegen die Folie bzw. den Film gedrückt wird.

Die Beobachtung der unterschiedlichen Helligkeitsabstufungen bei wechselnder Beschichtungs- oder Papierqualität kann von Auge erfolgen. Als besonders zweckmäßig hat es sich aber erwiesen, wenn die Beobachtung mit einer CCD-Kamera erfolgt und das Bild derselben elektronisch analysiert wird.

Durch das erfindungsgemäße Verfahren kann nicht nur das Papier aus laufender Produktion überwacht werden. Es kann vielmehr auch festgestellt werden, wie sich die Qualität des Papiers verbessert oder verschlechtert, wenn Parameter der Produktion geändert werden. Weiter kann geprüft werden, wie sich die Qualität mit der Qualität von früheren Produktionen oder mit einem Standard vergleicht. Die Prüfung kann sowohl im Labor als auch in der Produktion erfolgen.

Die Punkte können auch ohne transparenten Träger, z. B. Letra-Set aufgebracht werden.

Die Erfindung wird im folgenden anhand einer vorteilhaften Ausführungsform unter Bezugnahme auf die beigefügten Zeichnung beispielsweise beschrieben. Es zeigen:
- Fig. 1: die Streuung von Licht in beschichtetem Papier, das bedruckt ist;
- Fig. 2: die Punktvergrößerung bei unterschiedlicher Dicke der Beschichtung;
- Fig. 3: im Detail den prinzipiellen Aufbau der Meßanordnung;
- Fig. 4: schematisch die gesamte Meßanordnung.
- Fig. 5: einen Querschnitt durch die für die Erfindung wesentlichen Teile einer anderen Vorrichtung, mit der das Verfahren der Erfindung ausgeführt werden kann; und
- Fig. 6: einen Querschnitt durch Teile der Vorrichtung von Fig. 5.

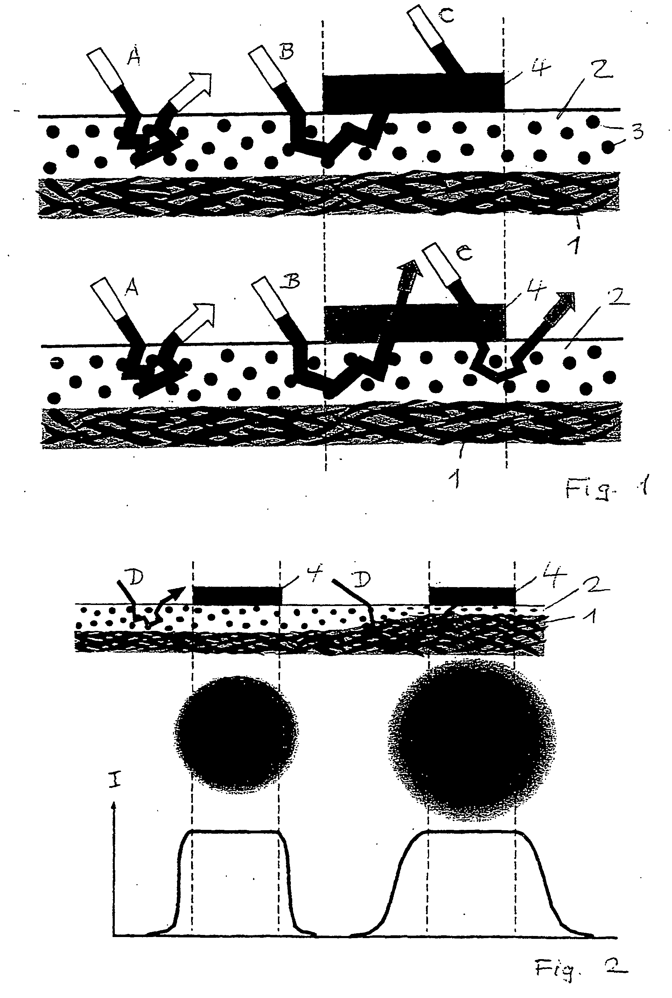

Wie dies in Fig. 1 gezeigt ist, ist auf einem aus Fasern bestehenden Papiersubstrat 1 eine teilweise lichtdurchlässige Beschichtung 2 aufgebracht, die zum Beispiel Pigmente 3 aufweist. Lichtstrahlen A, B und C werden unterschiedlich gestreut. In Fig. 1 ist dabei im oberen Teil ein schwarzer lichtundurchlässiger gedruckter Punkt 4 gezeigt, während im unteren Teil dieser gedruckte Punkt 4 teildurchlässig für Licht ist, wie etwa cyanfarben. Der Lichtstrahl A wird in beiden Fällen aus der Beschichtung 2 wieder herausgestreut und trägt damit zur Helligkeit des Bildes an dieser Stelle bei. Der Lichtstrahl B wird bei der oberen Darstellung mit einem schwarzen Druckpunkt unter diesen Druckpunkt 4 gestreut und kann daher die Beschichtung 2 nicht wieder verlassen. Es wird daher um den Druckpunkt 4 herum ein etwas verschwommener dunkler Bereich auftreten, d.h. der Punkt 4 wird größer erscheinen, als er tatsächlich ist. Nicht nur Lichtstrahlen C, die direkt auf den Druckpunkt 4 fallen, werden also ausgelöscht, sondern auch Lichtstrahlen, die nahe beim Punkt 4 in die Beschichtung 2 eintreten.

Bei der unteren Ausführungsform von Fig. 1 werden die Lichtstrahlen, die in den Druckpunkt 4 eintreten, nicht völlig ausgelöscht, sondern geschwächt. Dies führt dazu, daß der Lichtstrahl B nur geschwächt aus dem Punkt 4 heraustritt, so daß der Rand des Punktes 4 dunkler erscheint. Andererseits wird der Lichtstrahl C, wenn auch abgeschwächt, aus der Beschichtung 2 heraustreten, was auch dazu beiträgt, daß dieser Punkt 4 verschwommen erscheint.

Bei unterschiedlichen Papierqualitäten bzw. Beschichtungsqualitäten wird nun die Punktvergrößerung unterschiedlich sein, wie dies in Fig. 2 dargestellt ist. Beim linken Punkt 4 wird der Lichtstrahl D noch aus der Beschichtung 2 herausgestreut, so daß an dieser Stelle die Beschichtung 2 bei der Betrachtung noch hell erscheint. Bei dem rechten Punkt 4 ist aber die Beschichtung 2 dünner, so daß der Lichtstrahl D absorbiert wird. Der rechte Punkt 4 erscheint daher größer, wie dies im unteren Teil von Fig. 2 durch die Abbildungen der Punkte bzw. den Verlauf I der Intensität der Lichtabsorption dargestellt wird.

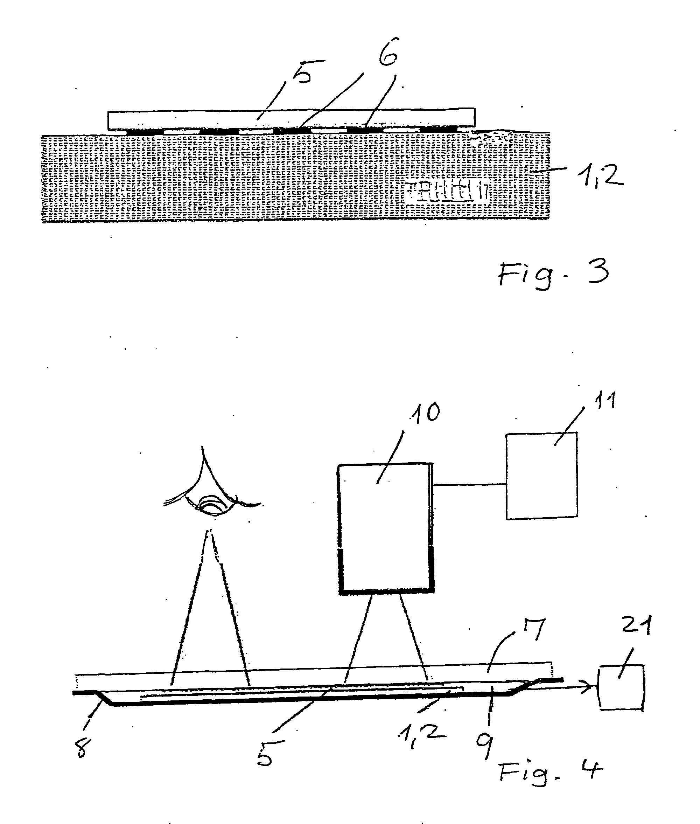

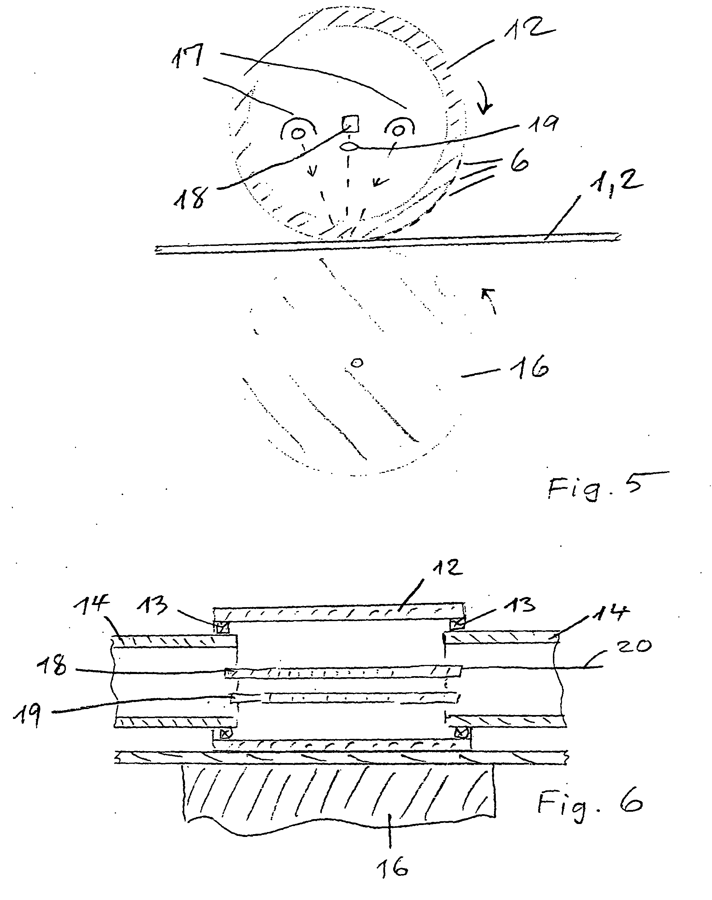

Unterschiedliche Papier- und Beschichtungsqualitäten können nun erfindungsgemäß durch eine Vorrichtung bestimmt werden, die im Zusammenhang mit den Fig. 3 und 4 kurz beschrieben werden soll. Auf dem Papier 1,2 (in den Fig. 3 und 4 wird zwischen Papierfasern 1 und Beschichtung 2 nicht unterschieden) ist ein Film 5 aufgebracht, der mit Punkten 6 versehen ist. Dieser Film 5 wird, wie dies in Fig. 4 gezeigt ist, durch eine Glasplatte 7 abgestützt. Auf der Seite des Films 5, auf dem sich die Punkte 6 befinden, wird das Papier 1,2 angelegt und durch eine Membran 8 gegen den Film 5 gedrückt, indem im Raum 9 zwischen Glasplatte 7 und Membran 8 auch eine bei 21 angedeutete Vakuumpumpe ein Unterdruck erzeugt und aufrechterhalten wird. Alternativ könnte von außen auf die Membran 8 ein Überdruck wirken (nicht gezeigt), oder es könnte das Papier 1, 2 durch einen gummielastischen Stempel (nicht gezeigt) gegen den Film gedrückt werden. Die Beobachtung kann dann entweder, wie dies in Fig. 4 links gezeigt ist, von Auge erfolgen. Eine andere Alternative besteht darin, die Beobachtung mit einer CCD-Kamera 10 vorzunehmen, was es dann erlaubt, in einer Einheit 11 eine elektronische Auswertung vorzunehmen.

In den Fig. 5 und 6 ist eine Ausführungsform gezeigt, bei der ebenfalls die vorstehend erwähnte elektronische Auswertung möglich ist. Das flächige Element ist dabei nicht eben, sondern eine Hohlwalze 12 aus transparentem Material, insbesondere Glas oder Kunststoff. Diese Walze ist, wie dies in Fig. 6 gezeigt ist, mit Lagern 13 auf Hohlrohren 14 gelagert und kann sich frei drehen. Das Papier 1,2 wird durch eine weitere Walze 16 insbesondere aus Gummi gegen die Walze 12 gedrückt. Die Walzen 12 und 16 werden entweder durch die Bewegung der Papierbahn 1,2 in Drehung in Richtung der Pfeile versetzt. Alternativ kann auch die Walze 16 motorisch angetrieben werden, um z. B. Einzelblätter für die Untersuchungen in die entsprechende Vorrichtung einzuziehen und zwischen den Walzen hindurch zu ziehen. In der Hohlwalze 12 sind Lichtquellen 17 und eine Zeile 18 von CCD-Elementen angeordnet. Die äußere Oberfläche der Walze 12 ist mit den bei 6 angedeuteten Rasterpunkten versehen. Das von den Lichtquellen 17 erzeugte, in der Fig. gestrichelt dargestellte Licht, das von der Papierbahn 1,2 reflektiert wird, durch eine bei 19 angedeutete Abbildungsoptik auf die Zeile 18 von CCD-Elementen abgebildet. Durch Auswertung der Signale der CCD-Elemente kann dann wie bei der vorherigen Ausführungsform die Papierqualität bestimmt werden. Wie dies in Fig. 6 gezeigt ist, dreht sich zwar die Walze 12. Die Zeile 18 von CCD-Elementen, die optische Abbildungseinrichtung 19 und die in Fig. 6 nicht gezeigten Lichtquellen 17 drehen sich nicht mit, sondern sind an den stationären Rohren 14 befestigt, durch die auch die Ableitung der Signale der Zeile 18 von CCD-Elementen über die Leitung 20 erfolgt. In ähnlicher Weise erfolgt auch die Energiezufuhr zu den Lichtquellen 17.

## Patentansprüche

1. Verfahren zum Bestimmen der Papierqualität für autotypischen Rasterdruck, bei dem ein feinverteiltes Muster von geometrischen Figuren auf das Papier aufgebracht wird, das Papier beleuchtet wird und das vom Papier reflektierte und gestreute Licht beobachtet wird, **dadurch gekennzeichnet, daß** das Muster auf einem optisch transparenten flächigen Element vorgesehen ist, dieses mit der Seite, auf der sich das Muster befindet, mit dem Papier flächig in Berührung gebracht wird, ohne daß ein Druck ausgeführt wird, das Papier gegen das flächige Element gedrückt wird und in dieser Anordnung von Papier und flächigem Element das vom Papier reflektierte und gestreute Licht beobachtet wird.

2. Verfahren nach Anspruch 1, **dadurch gekennzeichnet, daß** die geometrischen Figuren Punkte sind.

3. Verfahren nach Anspruch 1 oder 2, **dadurch gekennzeichnet, daß** das Papier durch ein gummielastisches Andrükkelement gegen das transparente flächige Element gedrückt wird.

4. Verfahren nach Anspruch 1 oder 2, **dadurch gekennzeichnet, daß** das Papier durch eine mit einer Druckdifferenz beaufschlagte Membran gegen das transparente flächige Element gedrückt wird.

5. Verfahren nach Anspruch 4, **dadurch gekennzeichnet, daß** die Druckdifferenz durch eine Druckgasquelle bewirkt wird.

6. Verfahren nach Anspruch 4, **dadurch gekennzeichnet, daß** die Druckdifferenz durch einen Unterdruck bewirkt wird.

7. Verfahren nach einem der Ansprüche 1 bis 3, **dadurch gekennzeichnet, daß** das transparente flächige Element eine transparente rotierende Walze ist, in der eine oder mehrere Lichtquellen und lichtempfindliche Elemente angeordnet sind, und daß das Papier durch eine weitere Walze mit gummielastischer Oberfläche gegen die durchsichtige Walze gedrückt wird.

8. Verfahren nach einem der Ansprüche 1 bis 7, **dadurch gekennzeichnet, daß** die Beobachtung des gestreuten Lichts mit CCD-Elementen erfolgt.

9. Verfahren nach einem der Ansprüche 1 bis 8, **dadurch gekennzeichnet, daß** die geometrischen Figuren ungefähr 15-70%, insbesondere ungefähr 20-50% der gesamten Beobachtungsfläche bedecken.

10. Verfahren nach einem der Ansprüche 1 bis 9, **dadurch gekennzeichnet, daß** die geometrischen Figuren schwarz sind.

11. Verfahren nach einem der Ansprüche 1 bis 10, **dadurch gekennzeichnet, daß** die geometrischen Figuren regelmäßig gerastert sind und ungefähr 40 bis 100 geometrische Figuren bzw. Linien von Figuren pro Zentimeter vorgesehen sind.

12. Verfahren nach einem der Ansprüche 1 bis 7 **dadurch gekennzeichnet, daß** das transparente flächige Element eine Folie oder einen photographischen Film aufweist, die bzw. der durch eine transparente Glas- oder Kunststoffplatte bzw. eine Walze abgestützt wird.

13. Verfahren nach einem der Ansprüche 1 bis 6, **dadurch gekennzeichnet, daß** die Beobachtung mit dem menschlichen Auge erfolgt.

14. Verfahren nach einem der Ansprüche 1 bis 6, **dadurch gekennzeichnet, daß** die Beobachtung mit einer CCD-Kamera erfolgt und das Bild derselben analysiert wird.

## Claims

1. A method of determining the paper quality for auto-typical halftone printing, in which a finely distributed pattern of geometric figures is applied to the paper, the paper is illuminated and the light reflected and scattered by the paper is observed, **characterized in that** the pattern is provided on an optically transparent surface element, which is brought in flat manner into contact with the paper on the side on which the pattern is located, without a print being performed the paper is pressed against the flat element, and in this arrangement of paper and flat element the light which is reflected and scattered is observed.

2. The method as claimed in claim 1, **characterized in that** the geometric figures are dots.

3. The method as claimed in claim 1 or 2, **characterized in that** the paper is pressed against the transparent flat element by a rubber-like resilient pressing element.

4. The method as claimed in claim 1 or 2, **characterized in that** the paper is pressed against the transparent flat element by a diaphragm to which a pressure difference is applied.

5. The method as claimed in claim 4, **characterized in that** the pressure difference is effected by a compressed gas source.

6. The method as claimed in claim 4, **characterized in that** the pressure difference is effected by a vacuum.

7. The method as claimed in one of claims 1 to 3, **characterized in that** the transparent flat element is a transparent rotating roll in which one or more light sources and light-sensitive elements are arranged, and **in that** the paper is pressed against the transparent roll by a further roll with a rubber-like resilient surface.

8. The method as claimed in one of claims 1 to 7, **characterized in that** the observation of the scattered light is carried out by CCD elements.

9. The method as claimed in one of claims 1 to 8, **characterized in that** the geometric figures cover approximately 15-70%, in particular approximately 20-50%, of the total observation area.

10. The method as claimed in one of claims 1 to 9, **characterized in that** the geometric figures are black.

11. The method as claimed in one of claims 1 to 10, **characterized in that** the geometric figures are arranged in a regular grid pattern and approximately 40 to 100 geometric figures or lines of figures are provided per centimeter.

12. The method as claimed in one of claims 1 to 7, **characterized in that** the transparent surface element has a film or a photographic film which is supported by a transparent glass or plastic plate or a roll.

13. The method as claimed in one of claims 1 to 6, **characterized in that** the observation is carried out by the human eye.

14. The method as claimed in one of claims 1 to 6, **characterized in that** the observation is carried out with a CCD camera and the image from the same is analyzed.

## Revendications

1. Procédé pour la détermination de la qualité de papier pour une impression à trame d'autotypie, dans lequel un modèle finement réparti de figures géométriques est appliqué sur le papier, le papier est éclairé et la lumière réfléchie et diffusée par le papier est observée, **caractérisé en ce que** le modèle est prévu sur un élément plat transparent à l'oeil nu, cet élément est mis en contact à plat avec le papier par le côté sur lequel se trouve le modèle, sans qu'une pression soit exercée, le papier est appuyé contre l'élément plat et la lumière réfléchie et diffusée par le papier est observée dans cet agencement de papier et d'élément plat.

2. Procédé selon la revendication 1, **caractérisé en ce que** les figures géométriques sont des points.

3. Procédé selon la revendication 1 ou 2, **caractérisé en ce que** le papier est pressé par un élément d'appui élastique comme le caoutchouc contre l'élément plat transparent.

4. Procédé selon la revendication 1 ou 2, **caractérisé en ce que** le papier est pressé contre l'élément plat transparent par une membrane alimentée avec une différence de pression.

5. Procédé selon la revendication 4, **caractérisé en ce que** la différence de pression est créée par une source de gaz de pression.

6. Procédé selon la revendication 4, **caractérisé en ce que** la différence de pression est réalisée par une dépression.

7. Procédé selon l'une quelconque des revendications 1 à 3, **caractérisé en ce que** l'élément plat transparent est un cylindre rotatif transparent, dans lequel sont disposés une ou plusieurs sources de lumière et des éléments sensibles à la lumière, et **en ce que** le papier est pressé par un autre cylindre avec la surface élastique comme du caoutchouc contre le cylindre transparent.

8. Procédé selon l'une quelconque des revendications 1 à 7, **caractérisé en ce que** l'observation de la lumière diffusée s'effectue à l'aide d'éléments CCD.

9. Procédé selon l'une quelconque des revendications 1 à 8, **caractérisé en ce que** les figures géométriques recouvrent environ 15 à 70%, en particulier environ 20 à 50% de la surface d'observation totale.

10. Procédé selon l'une quelconque des revendications 1 à 9, **caractérisé en ce que** les figures géométriques sont noires.

11. Procédé selon l'une quelconque des revendications 1 à 10, **caractérisé en ce que** les figures géométriques sont régulièrement tramées et environ 40 à 100 figures géométriques ou lignes de figures sont prévues par centimètre.

12. Procédé selon l'une quelconque des revendications 1 à 7, **caractérisé en ce que** l'élément plat transparent présente une feuille ou un film photographique, qui est soutenue par une plaque de verre ou de plastique transparente ou un cylindre.

13. Procédé selon l'une quelconque des revendications 1 à 6, **caractérisé en ce que** l'observation s'effectue à l'oeil humain.

14. Procédé selon l'une quelconque des revendications 1 à 6, **caractérisé en ce que** l'observation s'effectue avec une caméra CCD et l'image de celle-ci est analysée.
